# EUROPEAN PATENT SPECIFICATION

(11) **EP 2 558 065 B1**
(45) Date of publication and mention of the grant of the patent: **08.06.2016**
(21) Application number: 11716854.2
(22) Date of filing: 05.04.2011
(51) Int. Cl.: A61K 9/00, A61K 31/198, A61K 47/26

(54) **EFFERVESCENT PHARMACEUTICAL COMPOSITIONS CONTAINING N-ACETYLCYSTEINE**
PHARMAZEUTISCHE BRAUSEZUSAMMENSETZUNGEN MIT N-ACETYLCYSTEIN
COMPOSITIONS PHARMACEUTIQUES EFFERVESCENTES CONTENANT DE LA N-ACÉTYLCYSTÉINE

(30) Priority: 13.04.2010 IT MI20100615
(43) Date of publication of application: 20.02.2013
(73) Proprietor: Alpex Pharma SA, 6805 Mezzovico (CH)
(72) Inventor: STROPPOLO, Federico, CH-6805 Mezzovico (CH); ARDALAN, Shahbaz, CH-6900 Massagno (CH)
(74) Representative: Longoni, Alessandra
(86) International application number: PCT/EP2011/055287
(87) International publication number: WO 2011/128230

(56) References cited:
- WO-A1-00/59500
- WO-A1-2010/090612
- GB-A- 2 192 790

## Description

The present invention relates to an effervescent pharmaceutical composition containing N-acetylcysteine and, more particularly, relates to an effervescent pharmaceutical composition containing N-acetylcysteine and sucralose, as defined in the claim.

N-acetylcisteine, commonly known also as NAC, is an active ingredient used for years mostly as a mucolytic. One of the main problems related to the formulation of NAC in soluble pharmaceutical forms, such as effervescent or mouth-soluble tablets and granules, is its unpleasant taste which is difficult to mask especially in high dosage forms (600 mg). Furthermore, the unpleasant taste of NAC is connected to its degradation products (mainly sulphides and disulphides) and it is then much more pronounced when the pharmaceutical form is stored for a long time especially at high room temperatures, as often happens during the hottest seasons or in regions where the climate is constantly warm.

Usually, the use of sweeteners and flavouring agents is able to cover, more or less effectively, the unpleasant taste of NAC in low dosage compositions but when the NAC amount is higher, it is impossible to obtain the same effectiveness in masking the unpleasant taste, except with the use of too large amounts of sweeteners and flavouring agents.

The need to use large amounts of excipients leads to the obtainment of pharmaceutical compositions that are difficult to prepare, pack and store. Moreover, the use of large amounts of sweetener can be a problem for many patients (for example for cavities or diet problems) or it can be even contra-indicated in the presence of other diseases (for example diabetes).

According to our knowledge, the only formulation of NAC containing a limited amount of excipients is the formulation into swallowable tablets described in WO 00/59500. These swallowable tablets contain 80-95% by weight of NAC and do not show, if not in a minimum part, the problem of masking the unpleasant taste because they should not need to be dissolved either in water or in the mouth.

However, the size of a tablet with a daily dosage (600 mg) is still considerable (about 750 mg) and it is not particularly suitable for the swallowing, especially in aged patients or for patients with breath diseases.

Mouth-soluble tablets of NAC contain large amounts of excipients in order to obtain an effective taste masking.

EP 0 339 508 describes mouth-soluble formulations containing NAC, alkaline bicarbonates, carbohydrates and fruit flavourings. The amount of carbohydrates is from 20:1 to 50:1 by weight compared with NAC.

EP 0 839 528 describes mouth-soluble compositions of NAC in which the taste masking is obtained using cyclodextrins. Although, the compositions also contain an amount from 2 to 15 parts of carbohydrates, preferably from 5 to 10 parts by weight compared with NAC. Even low dosage formulations (100-200g of NAC) have a total weight of at least 2 g.

The problem of excipient amount remains also in effervescent formulations because a suitable amount of effervescent couple (usually citric acid + sodium bicarbonate or equivalents thereof) should be added to sweeteners and flavouring agents, that are used for masking the unpleasant taste, in order to generate effervescence upon contact with water.

For example, GB2192790 describes effervescent formulations comprising from 6 to 32% by weight of NAC, from 35 to 50% by weight of citric acid, from 26 to 37% by weight of sodium bicarbonate, from 1 to 1.5% by weight of aspartame and from 5 to 7% by weight of a flavouring agent in which the weight ratio between citric acid and sodium bicarbonate is from 1.2:1 to 1.4:1. The effervescent tablet containing 600 mg of NAC has a total weight of 1900 mg. Similar effervescent tablets are marketed as Fluimucil^{®} 600 and have a total weight of 1.8 g.

Other commercial effervescent tablets (Solmucol^{®} Toux Grasse 600) contain NAC, sorbitol, flavouring agent, sodium saccharin and other excipients with a total weight of 3.0 g.

There is then the need of effervescent formulations comprising NAC with a pleasant taste but with a reduced total weight (approximately not higher than 1.5 g) which makes easier and cheapest its preparation, packaging and storage.

We have now found that effervescent formulations of NAC comprising sucralose have a very pleasant taste that persists over time even after the storage under condition of high room temperature and after opening the package for use and also allow to reduce the total amount of excipients.

Described herein is an effervescent formulation comprising from 5 to 50% by weight of N-acetylcysteine, from 20 to 60% by weight of citric acid, from 20 to 50% by weight of sodium bicarbonate, from 0.5 to 1% by weight of sucralose and from 4 to 5% by weight of a flavouring agent.

The effervescent formulation object of the present invention allows the preparation of tablets comprising 600 mg of NAC with a total weight of about 1.5 g while maintaining a pleasant taste and increasing the stability of the tablet.

The characterizing feature of the effervescent formulation object of the present invention is the use of sucralose as sweetener.

Sucralose is preferably used in an amount from 0.6 to 0.8% by weight, more preferably in an amount from 0.65 to 0.7% by weight.

It is important to underline that sucralose is an artificial sweetener with a sweetness about 600 times more powerful than sugar, about 2 times more powerful than saccharin and about 3 times more powerful than aspartame, which use is known to mask the unpleasant taste of active pharmaceutical ingredients (see, for example, EP 0 371 584, EP 1 210 880 and WO 2006/03416). It is not known, however, that its use in effervescent formulations allows to remarkably decrease the excipients, particularly the effervescent couple and to increase the stability of the formulation.

The effervescent couple used for the formulations object of the present invention is the couple citric acid/sodium bicarbonate, conventionally the most used in pharmaceutical technology.

It is evident to the expert in the field that any other effervescent couple, conventionally usable in pharmaceutical technology, can be use in the formulations object of the present invention instead of the mixture citric acid/sodium bicarbonate provided that it ensures a suitable effervescence.

Even the ratio between citric acid and sodium bicarbonate or more generally, between acid and base in the effervescent couple is that conventionally used for the preparation of effervescent formulations.

Their amount can vary in the indicated ranges depending on the quantities of NAC contained in the formulation. Generally, for effervescent formulations comprising a large amount of NAC (40-50% by weight), the amount of citric acid and sodium bicarbonate or their equivalents will be nearest to the minimum values of the indicated ranges, that is about 20-30% by weight both for citric acid and for sodium bicarbonate. For effervescent formulations comprising a low amount of NAC (5-20% by weight) the amount of citric acid and sodium bicarbonate or their equivalents, will be nearest to the maximum values of the indicated ranges, that is about 40-60% by weight of citric acid and about 30-50% by weight of sodium bicarbonate.

Therefore, effervescent formulations comprising from 40 to 50% by weight of N-acetylcysteine, from 20 to 30% by weight of citric acid, from 20 to 30% by weight of sodium bicarbonate, from 0.5 to 1% by weight of sucralose and from 4 to 5% by weight of a flavouring agent are a preferred object of the present invention.

Effervescent formulations comprising from 5 to 20% by weight of N-acetylcysteine, from 40 to 60% by weight of citric acid, from 30 to 50% by weight of sodium bicarbonate, from 0.5 to 1% by weight of sucralose and from 4 to 5% by weight of a flavouring agent are a further preferred object of the present invention.

The flavouring agent used in the effervescent formulations object of the present invention can be any flavouring agent conventionally used in pharmaceutical technology such as orange flavour, lemon flavour, peppermint flavour, anise flavour, apricot flavour, etc. Orange or lemon flavour are preferably used.

The effervescent formulations object of the present invention can be prepared according to conventional techniques of mixing, granulation, drying and optionally compression that will be illustrated in details in the experimental section.

The formulations object of the present invention can be in the form of granules but more preferably of water-soluble tablets.

The tablets have a reduced weight, generally not higher than 1.5 g, from 3.5 and 5.0 mm of thickness and from 17.5 and 18.5 mm of diameter.

Described herein are effervescent formulations having the following quantitative composition (in brackets percentages by weight):

| | |
|---|---|
| N-acetylcysteine | 100 mg (6.67%) |
| Citric acid | 740 mg (49.33%) |
| Sodium bicarbonate | 580 mg (38.67%) |
| Sucralose | 10 mg (0.67%) |
| Orange flavour | 70 mg (4.67%) |
| Total weight | 1500 mg |
| N-acetylcysteine | 200 mg (13.33%) |
| Citric acid | 690 mg (46.00%) |
| Sodium bicarbonate | 530 mg (35.33%) |
| Sucralose | 10 mg (0.67%) |
| Orange flavor | 70 mg (4.67%) |
| Total weight | 1500 mg |

The invention relates to an effervescent formulation comprising:

| | |
|---|---|
| N-acetylcysteine | 600 mg (40.00%) |
| Citric acid | 400 mg (26.66%) |
| Sodium bicarbonate | 420 mg (28.00%) |
| Sucralose | 10 mg (0.67%) |
| Orange flavor | 70 mg (4.67%) |
| Total weight | 1500 mg |

The effervescent formulations object of the present invention have a pleasant taste and smell, better compared with the commercially available formulations as proved in palatability tests in comparison with Fluimucil^{®} 600 and Solmucol^{®} Toux Grasse 600.

Moreover the effervescent formulations object of the present invention are particularly stable even in conditions of prolonged permanence at high ambient temperatures such as those of summer period or of tropical and equatorial areas as demonstrated by the stability data at the following conditions:

| Temperature | 40°C | 30°C | 30°C | 25°C |
|---|---|---|---|---|
| Relative humidity | 75% | 75% | 65% | 60% |
| Time (months) | 6 | 18 | 18 | 18 |

The improved stability of the formulations object of the present invention is confirmed even after ten days by proofs of use simulating the opening and the closure of the package by the patient.

Thanks to this stability, the effervescent formulations object of the present invention can be also packed in tubes without requiring the use of blister.

In order to better illustrate the present invention, without however limiting it, the following examples are now given.

### Example 1

### Preparation of an effervescent granulate

NAC, citric acid, sodium bicarbonate, sucralose and orange flavor were sieved using a rotating sieve, transferred in a fluid bed granulator and granulated with purified water according to the standards set by the European Pharmacopeia.

At the end of the drying/cooling phase, the granulate was sieved using a turbulence sieve and then transferred into a bin blender and blended for 10-20 minutes to guarantee the homogeneity.

### Reference Example 2

### Preparation of effervescent tablets

A granulate prepared as described in example 1, was compressed in a tabletting machine using punches with 18 mm diameter obtaining effervescent tablets having the following composition:

| | |
|---|---|
| N-acetylcysteine | 200 mg |
| Citric acid | 690 mg |
| Sodium bicarbonate | 530 mg |
| Sucralose | 10 mg |
| Orange flavor | 70 mg |
| Total weight | 1500 mg |

The resultant tablets were packed in polypropylene tubes containing 10 tablets and plugged with a polypropylene cap containing a desiccant.

### Example 3

### Preparation of effervescent tablets

By working in a similar way as described in example 2, effervescent tablets having the following composition were prepared:

| | |
|---|---|
| N-acetylcysteine | 600 mg |
| Citric acid | 400 mg |
| Sodium bicarbonate | 420 mg |
| Sucralose | 10 mg |
| Orange flavor | 70 mg |
| Total weight | 1500 mg |

The resultant tablets were packed in polypropylene tubes containing 10 tablets and plugged with a polypropylene cup containing a dessicant.

### Example 4

### Palatability tests

Effervescent tablets obtained as described in example 3 were compared with effervescent tablets of NAC (600 mg) commercially available as Fluimucil^{®} 600 and Solmucol^{®} Toux Grasse 600.

Tests were conducted on 8 healthy volunteers.

A tablet of each formulation was dissolved in water (200 ml) at 20°C.

Each volunteer was requested to independently evaluate the following parameters:
disintegration time
smell of the tablet
smell of the solution
taste of the solution
aftertaste
assigning a score from1 to 8 according to the following criteria:
   1 = poor
   2 = mild
   3 = good
   4 = excellent

The results are reported in the following tables:

**table 1 - palatability of a tablet according to the invention (example 3)**

| Subject | A | B | C | D | E | F | G | H | total |
|---|---|---|---|---|---|---|---|---|---|
| Disintegration time | 4 | 8 | 4 | 4 | 8 | 4 | 2 | 4 | 38 |
| Smell of the tablets | 4 | 4 | 8 | 4 | 2 | 4 | 8 | 4 | 38 |
| Smell of the solution | 4 | 8 | 4 | 2 | 4 | 2 | 4 | 8 | 36 |
| Taste of the solution | 8 | 8 | 8 | 8 | 4 | 4 | 8 | 8 | 56 |
| Aftertaste | 8 | 8 | 8 | 8 | 4 | 8 | 8 | 8 | 60 |
| Total score | 28 | 36 | 32 | 26 | 22 | 22 | 30 | 32 | 228 |

**table 2 - palatability of a Fluimucil^{®} 600 tablet**

| Subject | A | B | C | D | E | F | G | H | total |
|---|---|---|---|---|---|---|---|---|---|
| Disintegration time | 2 | 1 | 2 | 1 | 1 | 2 | 1 | 1 | 11 |
| Smell of the tablets | 2 | 2 | 4 | 4 | 4 | 8 | 4 | 4 | 32 |
| Smell of the solution | 4 | 4 | 2 | 8 | 4 | 8 | 4 | 2 | 36 |
| Taste of the solution | 4 | 2 | 4 | 4 | 2 | 4 | 2 | 2 | 24 |
| Aftertaste | 2 | 1 | 2 | 1 | 2 | 1 | 1 | 1 | 11 |
| Total score | 14 | 10 | 14 | 18 | 13 | 23 | 12 | 10 | **114** |

**table 3 - palatability of a Solmucol^{®} Toux Grasse 600 tablet**

| Subject | A | B | C | D | E | F | G | H | total |
|---|---|---|---|---|---|---|---|---|---|
| Disintegration time | 4 | 2 | 4 | 2 | 2 | 4 | 4 | 4 | 26 |
| Smell of the tablets | 4 | 4 | 8 | 4 | 4 | 4 | 4 | 4 | 36 |
| Smell of the solution | 8 | 4 | 4 | 4 | 2 | 8 | 4 | 4 | 38 |
| Taste of the solution | 4 | 8 | 4 | 4 | 2 | 1 | 1 | 4 | 28 |
| Aftertaste | 4 | 2 | 1 | 4 | 1 | 2 | 2 | 4 | 20 |
| Total score | 24 | 20 | 21 | 18 | 11 | 19 | 15 | 20 | **148** |

In the following table are reported the compared total score.

**table 4 - palatability - score comparison**

| Formulation | Example 3 | Fluimucil^{®} | Solmucol^{®} | (%) of evaluation in respect to the best result |
|---|---|---|---|---|
| Disintegration time | 38 | 11 | 26 | +**31**% |
| Smell of the tablets | 38 | 32 | 36 | **+5%** |
| Smell of the solution | 36 | 36 | 38 | *-5%* |
| Taste of the solution | 56 | 24 | 28 | **+50%** |
| Aftertaste | 60 | 11 | 20 | **+67%** |
| Total score | 228 | 114 | 148 | **+35%** |

The results clearly show that the formulation of the invention, despite the lower content of excipients (-17% than Fluimucil^{®} and -50% than Solmucol^{®}), has a significantly better flavor and smell than the compared products.

## Claims

1. An effervescent formulation having the following quantitative composition:
| | |
|---|---|
| N-acetylcysteine | 600 mg |
| Citric acid | 400 mg |
| Sodium bicarbonate | 420 mg |
| Sucralose | 10 mg |
| Orange flavour | 70 mg |
| Total weight | 1500 mg |

## Patentansprüche

1. Eine sprudelnde Formel mit folgender quantitativer Zusammensetzung:
| | |
|---|---|
| N-Acetylcystein | 600 mg |
| Zitronensäure | 400 mg |
| Natron | 420 mg |
| Sucralose | 10 mg |
| Orangenaroma | 70 mg |
| Gesamtgewicht | 1.500 mg |

## Revendications

1. Formulation effervescente ayant la composition quantitative suivante :
| | |
|---|---|
| N-acétylcystéine | 600 mg |
| Acide citrique | 400 mg |
| Bicarbonate de sodium | 420 mg |
| Sucralose | 10 mg |
| Parfum orange | 70 mg |
| Poids total | 1 500 mg |
